# FASCICULE DE BREVET EUROPEEN

(11) **EP 1 163 364 B1**
(45) Date de publication et mention de la délivrance du brevet: **08.10.2008**
(21) Numéro de dépôt: 00910978.6
(22) Date de dépôt: 17.03.2000
(51) Int. Cl.: C12Q 1/48

(54) **PROCEDE DE CRIBLAGE METTANT EN JEU LA MGDG SYNTHASE**
SCREENINGVERFAHREN UNTER VERWENDUNG DER MGDG-SYNTHASE
SCREENING METHOD INVOLVING MGDG SYNTHASE

(30) Priorité: 19.03.1999 FR 9903434
(43) Date de publication de la demande: 19.12.2001
(73) Titulaire: COMMISSARIAT A L'ENERGIE ATOMIQUE, 75015 Paris (FR); CENTRE NATIONAL DE LA RECHERCHE SCIENTIFIQUE (CNRS), 75794 Paris Cedex 16 (FR)
(72) Inventeur: MARECHAL, Eric, F-38000 Grenoble (FR); BLOCK, Maryse, F-38640 Claix (FR); JOYARD, Jacques, F-38240 Meylan (FR); DOUCE, Roland, F-38000 Grenoble (FR)
(74) Mandataire: Goulard, Sophie
(86) Numéro de dépôt international: PCT/FR2000/000658
(87) Numéro de publication internationale: WO 2000/056919

(56) Documents cités:
- E MAR¹CHAL, C MIÈGE, M A BLOCK, R DOUCE, J JOYARD: "The Catalytic Site of Monogalactosyldiacylglycerol Synthase from Spinach Chloroplast Envelope Membranes" JOURNAL OF BIOLOGICAL CHEMISTRY, vol. 270, no. 11, 17 mars 1995 (1995-03-17), pages 5714-5722, XP002123831 cité dans la demande
- E MAR¹CHAL, M A BLOCK, J JOYARD, R DOUCE: "Kinetic Properties of Monogalactosyldiacylglycerol Synthase from Spinach Chloroplast Envelope Membranes" JOURNAL OF BIOLOGICAL CHEMISTRY, vol. 269, no. 8, 25 février 1994 (1994-02-25), pages 5788-5798, XP002123832 cité dans la demande
- MCFADDEN G I ET AL: "PLASTID IN HUMAN PARASITES" NATURE,GB,MACMILLAN JOURNALS LTD. LONDON, vol. 381, page 482 XP002057546 ISSN: 0028-0836 cité dans la demande
- M SHIMOJIMA, H OHTA, A IWAMATSU, T MASUDA, Y SHIOI, K-I TAKAMIYA: "Cloning of the gene for monogalactosyldiacylglycerol synthase and its evolutionary origin" PROCEEDINGS OF THE NATIONAL ACADEMY OF SCIENCES OF USA, vol. 94, janvier 1997 (1997-01), pages 333-337, XP002123833 cité dans la demande
- AWAI ET AL: "Two types of MGDG synthase genes, found widely in both 16:3 and 18:3 plants, differentially mediate galactolipid syntheses in photosynthetic and nonphotosynthetic tissues in Arabidopsis thaliana" PROC.NATL.ACAD.SCI.USA, vol. 98, no. 19, 11 septembre 2001 (2001-09-11), pages 10960-10965,
- MIÈGE ET AL: "Biochemical and topological properties of type A MGDG synthase, a spinach chloroplast envelope enzyme catalysing the synthesis of both prokaryotic and eukaryotic MGDG" EUR.J.BIOICHEM. FEBS, vol. 265, 1999, pages 990-1001,

## Description

La présente invention est relative à un procédé de criblage et de sélection d'antiparasitaires (parasites du phyllum des apicomplexes) et/ou d'herbicides.

Les apicomplexes sont des parasites unicellulaires responsables de maladies parmi les plus graves pour l'espèce humaine : le paludisme, la première maladie mortelle dans le monde, et la toxoplasmose, une des deux infections opportunistes les plus fréquentes chez les malades du SIDA. Ces maladies infectieuses progressent alors qu'aucun traitement ne permet aujourd'hui d'éradiquer les parasites qui les provoquent : *Plasmodium* qui se niche dans les cellules hépatiques et les globules rouges des malades du paludisme, et *Toxoplasma* qui envahit entre autre le cerveau des malades de la toxoplasmose.

En effet, selon l'Organisation Mondiale de la Santé (OMS), le paludisme touche plus de 500 millions d'êtres humains et provoque 2,5 millions de morts par an. Le paludisme tue la moitié des enfants de moins de 5 ans en Afrique. 40 % de la population mondiale se trouve dans des zones impaludées, et ces zones progressent chaque année. Les traitements pesticides ont provoqué la résistance des moustiques vecteurs du parasite (les anophèles), et le parasite lui-même (4 espèces de *Plasmodium,* dont *Plasmodium falciparum* pour 95 % des cas) résiste de plus en plus aux traitements connus (en particulier les dérivés des chloroquines). Selon les estimations, le paludisme est la première ou la deuxième (après les diarrhées) maladie mortelle dans le monde. Le coût direct et indirect du paludisme en Afrique est passé de 800 millions de dollars en 1987 à plus de 2 milliards de dollars en 1998. La résistance aux traitements, et la progression des zones impaludées font de ce fléau un enjeu majeur du XXI siècle.

Selon le *National Institute of Health* (NIH), la toxoplasmose est la première infection cérébrale chez les malades du SIDA. Le parasite *(Toxoplasma gondii)* est commun et l'on peut considérer qu'une personne sur deux a été infectée, soit en mangeant de la viande mal cuite, soit au contact de chats domestiques. La toxoplasmose n'est grave que chez les personnes fragiles, en particulier les foetus humains et les malades du SIDA. Dans le cas du SIDA, les patients présentant un taux en CD4⁺<100/mm³ développent les symptômes de la toxoplasmose, en général par réactivation d'une infection antérieure. Les traitements connus (les plus communs sulfadiazine et pyriméthamine, mais aussi clindamycine, azithromycine, clarithromycine, dapsone et atavaquone), doivent parfois être prescrits indéfiniment car bien que létales pour le parasite *in vitro,* ces substances n'éliminent pas toujours le parasite de l'organisme. Ces traitements étant parfois incompatibles avec la trithérapie, la prophylaxie est difficile. Dans le combat qu'il reste à mener contre le SIDA, il est donc fondamental de rechercher de nouveaux traitements susceptibles d'éradiquer *Toxoplasma.*

D'autres apicomplexes tels que ceux du genre *Eimeria,* sont responsables de la coccidiose chez les oiseaux et les bovins.

Depuis peu, l'on sait que ces parasites présentent des structures subcellulaires végétales (McFadden et al., Nature, 1996, 384, 482 ; Köhler et al., Science, 1997, 275, 1485-1489), dénommées apicoplastes.

Ces Auteurs ont identifié par hybridation *in-situ,* le plaste qui contient un ADN de 35 kb chez *Toxoplasma gondii :* c'est un organite limité par 4 membranes, proche dans l'évolution de celui des algues vertes. Très vite, ce plaste a été présenté comme une faille des parasites apicomplexes (Fichera et Roos, Nature, 1996, 390, 407-409). Ces Auteurs ont en particulier montré que certains antibiotiques, tels que les fluoroquinolones et les macrolides inhibent les ADN gyrases procaryotes et bloquent la réplication de cet ADN de 35 kb, qui semble nécessaire à la survie du parasite. Plus récemment, Waller et al. (PNAS, 1998, 95, 12352-12357) ont montré que ce plaste contenait une protéine connue pour synthétiser les acides gras dans les chloroplastes végétaux, l'acyl-carrier-protéine ou ACP. Le précurseur de l'ACP contient une séquence de transit de type chloroplastique, qui permet à la protéine (l'ACP mature, ou un marqueur fluorescent de type GFP fusionné avec la séquence de transit du précurseur de l'ACP) d'être intégrée dans le plaste du parasite.

L'ACP n'est malheureusement pas spécifique du règne végétal, et on la trouve chez les bactéries, en particulier du tractus intestinal. Elle ne constitue donc pas une cible spécifique pour un médicament qui n'affecterait que les apicomplexes parasitant l'organisme.

Les Inventeurs se sont donc donnés pour but de pourvoir à une cible spécifique des parasites apicomplexes, pour sélectionner de nouveaux médicaments efficaces contre lesdits parasites apicomplexes.

Ils ont maintenant trouvé que la MGDG synthase (enzyme capitale pour la biogénèse de l'enveloppe des plastes) peut être une cible de choix pour des principes actifs contre *Plasmodium* (paludisme), *Toxoplasma* (toxoplasmose) et *Eimeria* (coccidiose) et pour des herbicides.

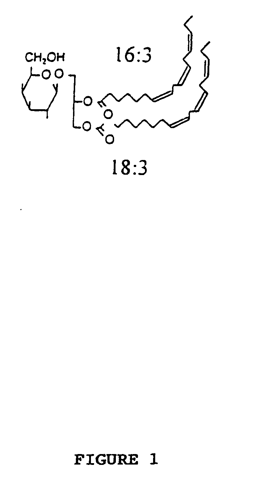

En effet, le MGDG (monogalactosyldiacylglycérol, figure 1) est connu dans tous les plastes analysés à ce jour : il est le lipide le plus abondant des membranes plastidiales ( > 50 % des glycérolipides), vital à la biogénèse des plastes et à la survie des cellules, et n'existe pas dans les autres systèmes membranaires, en particulier dans les cellules animales (Douce, Science, 1974, 183, 852-853) ; sa biosynthèse est catalysée dans l'enveloppe par une UDP-galactose:1,2-diacylglycérol 3-β-D-galactosyltransférase (EC 2.4..1.46) appelée aussi MGDG synthase, selon la réaction suivante :

1,2-diacylglycérol + UDP-galactose → UDP + 1,2-diacyl-3-β-D-galactopyranosyl-*sn*-glycérol.

La présente invention a pour objet l'utilisation d'une MGDG synthase pour la sélection ou le criblage de produits inhibiteurs de l'activité de la MGDG synthase, aptes à servir de principes actifs contre les parasites apicomplexes et notamment ceux responsables du paludisme, de la toxoplasmose et de la coccidiose.

La présente invention a également pour objet l'utilisation d'une membrane plastidiale isolée de plante, pour la sélection ou le criblage de produits inhibiteurs de l'activité de la MGDG synthase, aptes à servir de principes actifs contre les parasites apicomplexes et notamment ceux responsables du paludisme, de la toxoplasmose et de la coccidiose.

La présente invention a également pour objet l'utilisation d'une MGDG synthase pour la sélection ou le criblage de produits inhibiteurs de l'activité de la MGDG synthase, aptes à servir d'herbicides.

La présente invention a, en outre, pour objet l'utilisation d'une membrane plastidiale isolée de plante, pour la sélection ou le criblage de produits inhibiteurs de l'activité de la MGDG synthase, aptes à servir d' herbicides.

La présente invention a également pour objet un procédé de criblage et de sélection d'antiparasitaires apicomplexes et/ou d'herbicides, caractérisé en ce qu'il comprend :
- l'incubation d'une substance à tester avec une MGDG synthase et
- la mesure de l'activité spécifique enzymatique, après ladite incubation.

On définit l'inhibition de l'activité enzymatique par la diminution de l'activité d'au moins 50 %, comme un pourcentage de l'activité contrôle (activité de l'enzyme traitée comme l'essai, mais en l'absence d'inhibiteur).

Conformément à l'invention, ladite MGDG synthase présente une activité spécifique initiale comprise de préférence entre 0,1 et 120 µmol de galactose incorporé/h/mg de protéine ; certaines MGDG synthases recombinantes peuvent présenter une activité spécifique supérieure à 120 µmol de galactose incorporé/h/mg de protéine.

Egalement, conformément à l'invention, la MGDG synthase est d'origine végétale (épinard, concombre ou Arabidopsis, notamment) et est sélectionnée dans le groupe constitué par les MGDG synthase A et les MGDG synthase B purifiées ou recombinantes.

Conformément audit procédé, l'incubation MGDG synthase/substance à tester est réalisée dans un milieu d'incubation contenant un tampon ajusté à un pH compris entre 6 et 9 (MOPS-NaOH, Tris-HCl, KH₂PO₄/K₂HPO₄, CAPS de 10 à 250 mM), en présence de détergents (CHAPS de 3 à 6 Mm ou LDAO), d'un agent réducteur (DTT 1-10 mM ou β-mercaptoéthanol), de phosphatidylglycérol (0,1-2 mM) et d'un sel (KCl ou NaCl, 10-300 mM) ; de préférence, ledit tampon contient 50 mM de MOPS-NaOH, pH 7,8, 4,5 mM de CHAPS, 1,3 mM de phosphatidylglycérol, 1 mM de DTT, 250 mM de KH₂PO₄/K₂HPO₄ et 250 mM de KCl.

Également conformément à l'invention, l'activité enzymatique de la MGDG synthase est mesurée après constitution de micelles, conformément à la méthode décrite dans Maréchal et al. (J. Biol. Chem., 1994, 269, 5788-5798).

Conformément à l'invention, ledit parasite apicomplexe est sélectionné dans le groupe constitué par *Plasmodium, Toxoplasma* et *Eimeria.*

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés, dans lesquels :
- la figure 1 représente le MGDG (monogalactosyldiacylglycérol) ;

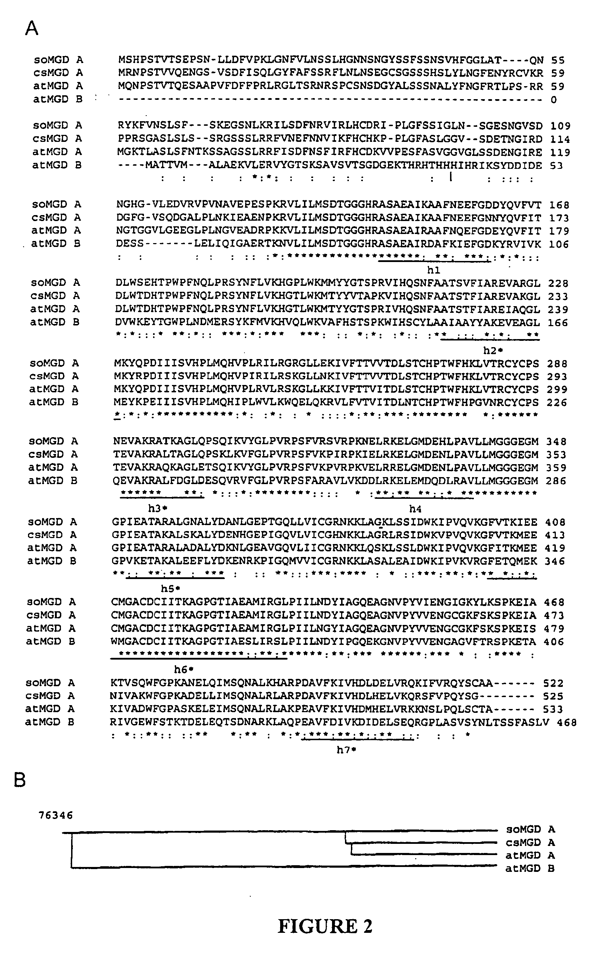

- la figure 2 est une comparaison de la MGDG synthase d'épinard, de concombre et d'Arabidopsis ; la figure 2A correspond à une comparaison des séquences en amino acides déduites des ADNc codant pour les différentes MGDG synthases ; dans cette figure atMGD A et atMGD B correspondent à des séquences issues *d'Arabidopsis thaliana,* csMGD A correspond à une séquence issue de *Cucumis sativa* et soMGD A correspond à une séquence issue de *Spinacia oleracea.* * et : représentent des symboles respectivement pour les amino acides identiques et les substitutions conservées ; h1 à h7 correspondent à 7 hélices-α putatives ; la figure 2B représente un arbre phylogénique des MGDG synthases matures ;
- la figure 3 correspond à l'identification du produit de la réaction de la *r*MGD A ; la figure 3A : séparation des lipides polaires par chromatographie en couche mince bi-dimensionnelle ; dans cette figure MGDG = monogalactosyldiacylglycérol ; DGDG = digalactosyldiacylglycérol ; TGDG = trigalactosyldiacylglycérol ; SL = sulfolipide ; PC = phosphatidylcholine ; PG = phosphatidylglycérol ; la figure 3B correspond à l'analyse des galactolipides synthétisés *in vitro* par la *r*MGD A ;
- la figure 4 illustre la localisation de la *r*MGD A dans *E*. *coli* ;
- la figure 5 correspond à la purification partielle de la *r*MGD A ; figure 5A : fractionnement par chromatographie hydroxyapatite-agarose ; figure 5B : analyse en SDS-Page de la fraction éluée à partir de la colonne hydroxyapatite-agarose.

Il doit être bien entendu, toutefois, que ces exemples sont donnés à titre d'illustration de l'objet de l'invention, dont ils ne constituent en aucune manière une limitation.

### Exemple 1 : Préparation d'une MGDG synthase, à partir d'une plante.

On solubilise et on purifie la MGDG **synthase** à partir de l'enveloppe des chloroplastes d'épinard, dans les conditions exposées dans Maréchal et al. (C.R. Acad. Sci. Paris, 1991, 313, III, 521-528 ; J. Biol. Chem., 1994, 269, 8, 5788-5798 ; J. Biol. Chem., 1995, 270, 11, 5714-5722).

Concrètement :
- on purifie les membranes d'enveloppe des chloroplastes d'épinard (voir Maréchal et al., J. Biol. Chem., 1995, précité).

On peut également obtenir les membranes d'enveloppe, conformément à la technique suivante : de manière plus précise, toutes les opérations sont réalisées à 0°-5°C. Les chloroplastes sont obtenus à partir de 3-4 kg de feuilles d'épinard (*Spinacia oleracea* L.) et purifiés par centrifugation isopycnique, à l'aide de gradients de Percoll. Les chloroplastes intacts purifiés sont lysés dans un milieu hypotonique et les membranes d'enveloppe sont purifiées à partir du lysat par centrifugation en gradient de saccharose.

Les membranes d'enveloppes obtenues sont stockées sous azote liquide, dans un milieu comprenant 50 mM de MOPS-NaOH, pH 7,8 et 1 mM de DTT (dithiothréitol).
- on solubilise et on purifie la MGDG synthase à partir des membranes d'enveloppe obtenues, comme précisé ci-dessus (voir Maréchal et al., J. Biol. Chem., 1995, précité).

On peut également obtenir de la MGDG synthase, à partir du concombre (Demande JP 98/014579 au nom de Kirin Brewery Co. Ltd).

Dans le cadre de la mise en oeuvre du procédé selon la présente invention, il est préférable d'utiliser une MGDG synthase présentant au moins une activité spécifique de 0,1 µmol de galactose incorporé/h/mg de protéine.

### Exemple 2 : Préparation d'une MGDG synthase recombinante et constructions pour sa surexpression dans E. coli.

### 1) Clonage et surexpression d'une MGDG de classe A dans E. coli.

### - Clonage de l'ADNc de MGDG synthase :

Un fragment de 1647 pb correspondant à la protéine mature de l'ADNc de MGDG de concombre (Shimojima et al., 1997) est utilisé comme sonde pour cribler une banque λgt11 obtenue à partir de feuilles d'épinard.

Avant le criblage, la présence d'un ARNm homologue est vérifiée par *Northern blot* à partir d'ARN total de feuilles d'épinard. 320 000 plaques sont cultivées sur E. *coli* Y1090 et transférées sur des membranes Hybond-N⁺. Les membranes sont préhybridées pendant 2h à 60°C dans une solution comprenant 2 x SSC, 5 x Denhardt's, 0,5 % SDS (w/v) et de l'ADN de sperme de saumon (0,1 mg.ml⁻¹).

L'hybridation est réalisée pendant 16h à 60°C dans le même réactif en présence d'1 ng d'ADN de concombre marqué au [α-³²P]dCTP. Les membranes sont lavées 3 fois 3 min à température ambiante dans du 2 x SSC, 0,1 % SDS (w/v) et 2 fois 15 min à 55°C, puis autoradiographiées.

Deux clones positifs sont ensuite purifiés par 3 niveaux de criblage. L'ADN de phage est extrait et digéré avec *EcoRI* ou *EcoRI* et *BamHl.* Les deux inserts d'ADNc sont sous-clonés dans le plasmide pBlueScript SK+ (digéré par *EcoRI* ou digéré par *EcoRI-BamHI),* pour le séquençage.

L'analyse du fragment de restriction et le séquençage montrent que les deux clones correspondent au même ADNc.

L'amplification par PCR avec des amorces adjacentes au site de clonage λgt11 révèle des inserts de 2,5 et 0,9 kb, respectivement. L'analyse de la séquence des inserts obtenus par PCR montre que l'insert de 0,9 kb est identique à l'extrémité 3' de l'insert de 2,5 kb.

En conséquence, l'insert le plus long est coupé à l'aide des enzymes de restriction *BamHI*/*EcoRI,* sous-cloné dans le plasmide pBlueScript SK+ (Stratagene) et séquencé.

La séquence obtenue, qui comprend 1851 nucléotides semble être une chimère. Elle contient une séquence de 807 nucléotides qui présente une homologie élevée avec l'extrémité codante en 3' de l'ADNc de la MGDG synthase de concombre, incluant le codon stop. Cet ADN tronqué est fusionné à son extrémité 5' avec une séquence d'ADN partielle (1044 nucléotides), homologue à des β-endoglucanases. L'extrémité 5' de l'ADNc de MGDG synthase est clonée par amplification rapide des extrémités d'ADNc (RACE) à l'aide du kit d'amplification Marathon (Clontech).

L'ADNc de feuilles d'épinard est préparé à partir d'ARNm polyA+ et utilisé comme matrice pour les amplifications PCR de l'extrémité 5' de l'ADNc de MGDG synthase, conformément aux instructions du fabricant. La spécificité de la réaction est due à l'amorce spécifique CTCATTTGAAGGGCAGTAGCACC (nucléotides 870 à 848) (SEQ ID NO:1) et par la PCR " hot start ".

Cette méthode permet de cloner un fragment de 1001 pb qui est ensuite sous-cloné dans le plasmide pBlueScript SK+ et séquencé sur les 2 brins dans 3 clones indépendants, de manière à être sûr que la Taq polymérase n'introduit aucune erreur.

Le fragment 5' RACE inclut un codon d'initiation identifiable et 131 nucléotides de la séquence en 5' non traduite. Le clone comprenant l'ADNc complet de la MGDG synthase est généré à partir de l'ADNc de feuilles d'épinard par PCR, en utilisant des amorces spécifiques des extrémités 3' et 5' de l'ADNc. L'amorce sens est la suivante : CACACAATATTTCCAATGTATACCCAC (nucléotides -82 à -57) (SEQ ID NO:2).

L'amorce anti-sens est la suivante : GATTATCATTTCCCCTCGCCCTGCC (nucléotides 1672 à 1648) (SEQ ID NO:3).

Le fragment d'ADN obtenu de 1765 pb est sous-cloné dans le plasmide pBlueScript SK+ au niveau du site de restriction *SmaI* et la séquence est vérifiée.

La séquence d'ADNc est plus petite que le transcrit (2,5 kb) détectée par une analyse en *Northern blot,* indiquant ainsi qu'elle n'est pas complète.
Les 2 techniques combinées (technique 5'-RACE et criblage d'une banque d'ADNc d'épinard) permettent d'obtenir une séquence de 1890 pb, qui inclut un cadre ouvert de lecture de 1569 pb, qui code pour une protéine de 522 acides aminés (57,5 kDa) (figure 2), qui appartient à la famille des MGDG synthases A.

L'analyse de la séquence en acides aminés montre que cette MGDG synthase A contient plus de résidus non polaires (56 %) que polaires (44 %), 9 résidus cystéine et 16 résidus histidine qui pourraient être impliqués dans la chélation des métaux ; cette protéine présente un point isoélectrique basique (pl = 9,16).

### 2) Extraction

### . extraction de la MGDG synthase recombinante (rMGD A)

toutes les opérations sont réalisées à 4°C. Un culot de bactéries recombinantes (34 mg de protéine), exprimant la MGDG synthase (7 mg de protéine) est remis en suspension dans 50 ml de milieu A (6 mM de CHAPS, 50 mM de MOPS-NaOH, pH 7,8, 1 mM de DTT) contenant 50 mM de KH₂PO₄/K₂HPO₄ et un mélange d'inhibiteurs de protéases (1 mM de PMSF ; 1 mM de benzamidine ; 0,5 mM d'acide caproïque). Après une lyse cellulaire par sonication répétée, la suspension est mélangée à 0°C dans de la glace) pendant 30 minutes. Le mélange est centrifugé pendant 15 min. à 243 000xg (Beckman L2, rotor SW 40). Le surnageant contenant les protéines solubilisées (16 mg) est chargé sur une colonne (Pharmacia C10/20, 25 ml de gel) hydroxyapatite-ultrogel (IBF-France), équilibrée avec un milieu A contenant 50 mM de KH₂PO₄/K₂HPO₄. Les protéines sont éluées, en utilisant un gradient de KH₂PO₄/K₂HPO₄ (50-275 mM) (dans un milieu A ; débit : 30 ml/h ; volume des fractions : 1,5ml). La MGDG synthase recombinante est éluée à 275 mM de KH₂PO₄/K₂HPO₄.

### 3) Surexpression de la MGDG synthase d'épinard dans E. coli

### - Matériel et méthodes

Deux formes matures de MGDG synthase sont surexprimées dans E. coli, en utilisant le plasmide pET-15b (Novagen) et un plasmide, dénommé pET-Y 3a, qui permet de surmonter le problème dû au fait que la séquence déduite de la MGDG synthase contient 22 résidus arginine, parmi lesquels 17 sont codés par AGG ou AGA, codons qui sont en fait peu utilisés chez *E. coli.* En effet, pET-Y 3a a été construit en insérant dans le plasmide pET-3a (Novagen), le gène arg U (ou DNA Y) codant pour l'ARN de transfert de l'arginine associé aux codons rares AGA/AGG.

Ces deux plasmides sont linéarisés avec *BamHl* et *Ndel.* Les fragments amplifiés par PCR sont générés à partir du clone d'ADNc complet. Le plasmide pET-15 est ligaturé avec un fragment codant les 417 résidus C-terminaux de l'enzyme, qui est amplifié par PCR à l'aide des amorces suivantes :
Amorce sens : GGAG*CATATG*GGGGTGAGTGATAATG (SEQ ID NO:4) et
Amorce anti-sens : GTTCT*GGATCC*TCAAGCAGCACAAGAGT (SEQ ID NO:5)
et digéré par les enzymes *BamHI* et *Ndel.*

Un autre fragment digéré par les enzymes *BamHl* et *Ndel, est* amplifié par PCR, à l'aides des amorces suivantes :
Amorce sens : CTTCA*CATATG*CTTAATTCCGGGGAGAG (SEQ ID NO:6) et
Amorce anti-sens: GTTCT*GGATCC*TCAAGCAGCACCGAGTA (SEQ ID NO:7),
codant les 424 résidus C-terminaux de l'enzyme, est sous-cloné au niveau du site de restriction *BamHI-Ndel* du plasmide pET-Y3.

La première construction permet l'expression d'une protéine de fusion étiquetée histidine (*h*MGD A), comprenant 437 résidus (48,24 kDa). La seconde construction permet l'expression d'une protéine de 425 acides aminés (*r*MGD A), incluant une méthionine d'initiation additionnelle, correspondant au codon ATG du site de restriction *BamHI.* Les protéines recombinantes sont exprimées dans *E*. *coli* BI.21(DE3). Les cultures bactériennes sont cultivées à 37°C, sous agitation vigoureuse (Certomat, 250 rpm), jusqu'à l'obtention d'une densité optique de 0,4 à 0,6. L'expression de la MGDG synthase recombinante est induite par l'addition de 0,4 mM d'IPTG dans le milieu et les cultures sont incubées 3 h à 25°C. Les bactéries sont rassemblées en culot par centrifugation (Eppendorf, 14000 x g, 10 min), et solubilisées dans un tampon A (MOPS 50 mM, pH 7,8, DTT 10 mM, EDTA 1 mM, benzamidine 1 mM, PMSF 1 mM et acide caproïque 0,5 mM) en présence ou en l'absence de Triton X-100 0,1 % ou dans un tampon A avec de l'urée 6M. Les fractions solubles et insolubles sont séparées par centrifugation (Airfuge, 115 000 x g, 15 min) et analysées sur SDS-PAGE (gel de polyacrylamide à 12 %). Les protéines sont détectées par une coloration au bleu de Coomassie.

L'hMGD est purifiée à homogénéité à partir des bactéries par chromatographie d'affinité à base d'un métal (NTA, Novagen), suivie d'un désalage à travers une colonne PD10 (Pharmacia), équilibrée dans un mélange comprenant de l'imidazole 5 mM, du NaCl 0,5 mM, du Tris-HCl 20 mM, pH 7,9, en présence d'urée 6M.

La protéine recombinante pure (1 mg) est utilisée pour obtenir un anticorps polyclonal de lapin (Eurogentec, Belgique). L'IgG est purifiée par chromatographie DEA-trisacryle M (IBF, France).

### - Résultats

Afin de minimiser l'effet de l'extrémité N-terminale de la séquence cible de chloroplaste, l'ADNc d'épinard est exprimé à partir du résidu leucine 99, qui correspond au site de clivage putatif du peptide signal du précurseur de la MGD A de concombre (Shimojima et al., PNAS, 1997, 94, 333-337).

En utilisant de l'UDP-[¹⁴C]-gal comme substrat, on peut mesurer l'activité de la MGDG synthase dans les extraits d'*E*. *coli* exprimant la rMGD A, après induction par IPTG : plus de 2 µmol de galactose sont incorporées/h/mg de protéine. L'activité déterminée dans les extraits d'E. *coli* contenant la protéine étiquetée à l'histidine hMGD A est du même ordre (1,3 µmol de galactose incorporé/h/mg de protéine).

Seulement une fraction négligeable de [¹⁴C]-galactose (moins de 0,1 µmol de galactose incorporé/h/mg de protéine) est observée dans les lipides d'E. *coli* avant l'induction par IPTG. De plus, aucune incorporation de [¹⁴C]-galactose n'est observée dans les bactéries contrôles, exprimant E37, une autre protéine d'enveloppe interne (Teyssier et al., Plant J., 1996, 10, 903-912). Après 3 h d'induction par IPTG, un extrait d'*E. coli* contenant la *r*MGD A surexprimée est incubé en présence d'UDP-C¹⁴C]-gal et les lipides sont extraits, pour analyser les produits de la réaction.

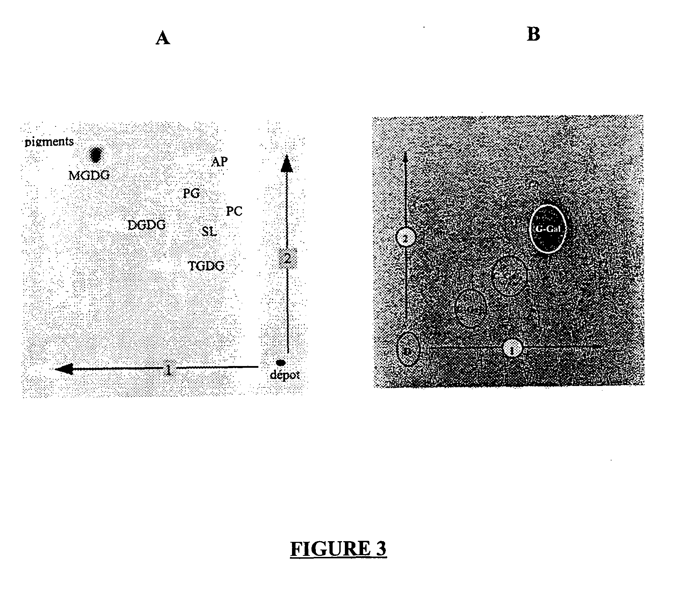

L'extrait lipidique est analysé par chromatographie en couche mince bi-dimensionnelle, en même temps que les lipides d'enveloppe ajoutés au mélange comme standard (Douce et al., In Methods in Plant Biochemistry, Lipids, Membranes and Aspects of Photobiology (Harwood et al. eds, 1990, 4, 71-103, Academic Press, Londres). La figure 3A montre qu'une seule tache radioactive co-migre avec le MGDG d'origine et est détectée par autoradiographie. Une caractérisation plus poussée du MGDG a été faite par l'analyse des groupes polaires par chromatographie papier bi-dimensionnelle ; dans ce cas là également, une seule tache radioactive est détectée par autoradiographie et co-migre avec le glycéryl-galactose obtenu après désacylation du MGDG d'enveloppe par hydrolyse alcaline douce (figure 3B).

Ces résultats montrent que le produit formé dans *E*. *coli* est effectivement du MGDG, qui est normalement absent dans les membranes d*'E. coli.* Aucun autre lipide contenant du galactose n'est formé, contrairement à ce que l'on observe après incubation de membranes d'enveloppe isolées, en présence d'UDP-[¹⁴C]-gal.

L'activité MGDG synthase est catalysée par une famille multigénique de protéines.

Grâce à des fractions protéiques membranaires très enrichies, le mécanisme biréactionnel de l'activité MGDG synthase a été étudié ainsi que sa sélectivité pour différentes espèces moléculaires de 1,2-diacylglycérol (Maréchal et al., J. Biol. Chem., 1994, 269, 5788-5798). Certaines propriétés structurales du site catalytique ont été élucidées : l'existence d'amino acides importants pour la catalyse (Cys, His, Lys) et l'association de l'enzyme à des métaux bivalents (Maréchal et al., J. Biol. Chem., 1995, 270, 5714-5722). La masse moléculaire fonctionnelle au cours de l'inactivation de la MGDG synthase a par ailleurs été déterminée par irradiation gamma : la masse moléculaire apparente de la MGDG synthase de l'enveloppe est de 97 ± 5 kDa. Le polypeptide MGDG synthase mature ayant une taille voisine de 45 kDa en gel dénaturant, il est probable que dans l'enveloppe, la MGDG synthase soit sous forme dimérique. Une masse moléculaire fonctionnelle de 114 ± 12 kDa a aussi été déduite par la même technique pour la MGDG synthase A recombinante purifiée. Ce résultat suggère que les MGDG synthases sont vraisemblablement des homodimères.

### Exemple 3 : Mesure de l'activité enzymatique, à partir de micelles.

L'activité de la MGDG synthase, est effectuée sur différents types d'échantillons, selon le modèle choisi : membrane plastidiale, fractions membranaires d'*E*. *coli* surexprimant une MGDG synthase recombinante (*r*MGD A, 0,7 µg protéine/test, enzyme préalablement extraite à partir d'une plante (voir exemple 1).

### . préparation des micelles

1,3 mM de phosphatidylglycérol (PG) et 160 µM de diacylglycérol (DAG) sont dissous dans du chloroforme. Après évaporation du solvant sous argon, 200 µl de milieu d'incubation contenant 50 mM de MOPS-NaOH, pH 7,8, 4,5 mM de CHAPS, 1 mM de DTT, 250 mM de KH₂PO₄/K₂HPO₄ et 250 mM de KCI sont ajoutés et le milieu est vigoureusement mélangé, de manière à remettre en suspension les lipides. 100 µl de fractions contenant la MGDG synthase, dans le milieu d'incubation, sont introduites, puis le milieu est à nouveau vigoureusement mélangé, puis maintenu 1 h à 20°C.

Cette procédure permet d'obtenir des micelles, conformément à Maréchal et al., 1994, précité.

### . réaction enzymatique

La réaction dans le mélange d'incubation est alors initiée par l'addition d'1 mM d'UDP-[¹⁴C]gal (37 Bq/µmol). Après 10 min. à 1 h, la réaction est stoppée par l'addition d'un mélange chloroforme/ méthanol (1 :2, v/v), les lipides sont extraits conformément à la méthode de Bligh et al (Can. J. Biochem. Physiol, 1959, 37, 911-917) et la radioactivité des galactolipides marqués est déterminée par comptage en scintillation liquide comme décrit dans Covès et al. (FEBS lett., 1986, 208, 401-406). L'activité est exprimée en µmol de galactose incorporé/h/mg de protéine.

Dans un échantillon riche en MGDG synthase, on peut obtenir une activité spécifique élevée, c'est-à-dire jusqu'à 115-120 µmol de galactose incorporé/h/mg de protéine, et même plus.

### 3) activité spécifique de la rMGD A soluble surexprimée

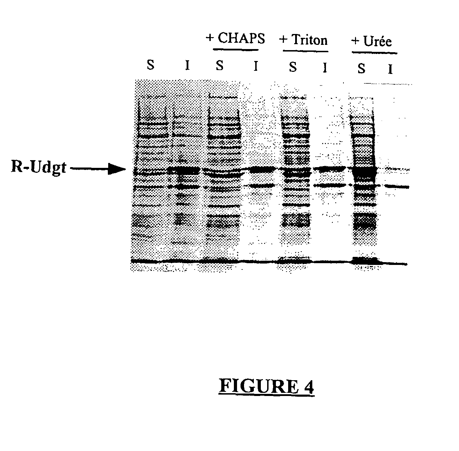

Lorsque l'on analyse l'expression de la MGDG synthase d'épinard dans *E*. *coli,* on observe que la plupart de la protéine (rMGD A) est insoluble et que des détergents (6 mM de CHAPS ou 1 % de Triton X-100) ne solubilisent que partiellement la protéine (figure 4). Par contre, presque toute la protéine surexprimée est solubilisée par de l'urée, indiquant que la plus grande partie de la MGDG synthase est présente dans des corps d'inclusion (figure 4). Dans cette fraction, l'activité de la MGDG synthase est très faible (0,03 µmol de galactose incorporé/h/mg de protéine). En fait, l'analyse par chromatographie d'hydroxyapatite des fractions d'*E. coli* solubilisées par le CHAPS montre que seulement une petite fraction (environ 0,1 %) de la protéine recombinante synthétisée par la bactérie est active. Les conditions expérimentales utilisées sont les mêmes que celles utilisées pour la MGDG synthase d'enveloppe (voir ci-dessus).

A la figure 4, l'expression de *r*MGD A est induite par 0,4 mM d'IPTG comme précisé dans Matériel et méthodes ci-dessus.

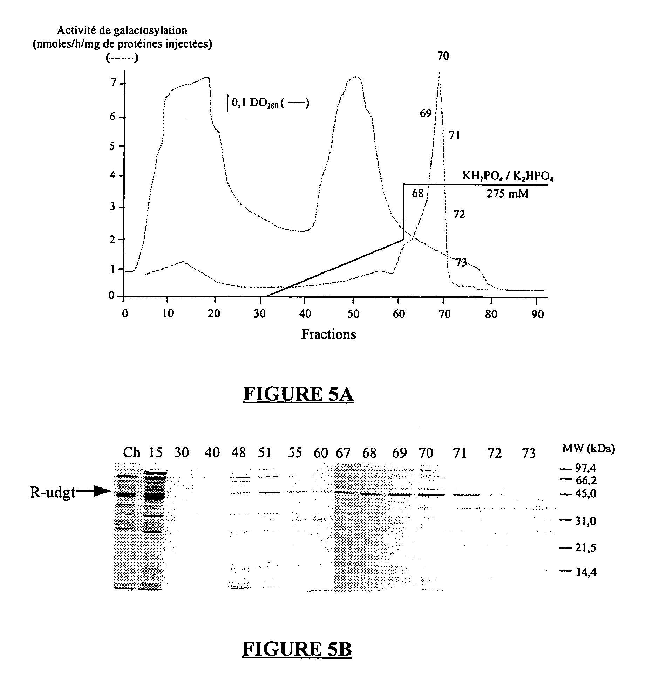

La plupart (50 à 80 %) de l'activité chargée par le haut de la colonne est retrouvée dans un pic étroit, élué avec du phosphate 275 mM (figure 5A). Dans ce pic, l'activité spécifique de la MGDG synthase est très élevée :115 µmol de galactose incorporé/h/mg de protéine.

L'analyse des polypeptides présents dans les différentes fractions montre qu'un polypeptide de 45 kDa, correspondant à la rMGD A, est présent dans les fractions actives, mais également dans le volume mort, dans lequel la plupart de la protéine est présente sous une forme inactive (figure 5B). Ceci montre que seulement une fraction (1 %) de la protéine solubilisée par le CHAPS est effectivement active.

Dans cette figure 5B, 20 µl de fraction éluée à partir de la colonne hydroxyapatite sont analysés par SDS-PAGE (gel de polyacrylamide à 12 %); les protéines sont détectées par coloration au bleu de Coomassie ; Ch : échantillon chargé par le haut de la colonne ; 15, 30, etc... : fractions éluées à partir de la colonne ; MW : marqueur de poids moléculaire (Biorad) ; la rMGD A est indiquée par une flèche et la rMGD A active se trouve uniquement dans les fractions 67 à 71.

### Exemple 4 : Comparaison des propriétés biochimiques de la MGD A surexprimée avec la MGDG synthase d'enveloppe de chloroplaste

L'analyse de l'activité de la MGDG synthase partiellement purifiée de chloroplastes de feuilles d'épinard (Maréchal et al., J. Biol. Chem., 1995, 270, 5714-5722) a démontré que le DTT peut protéger l'activité de l'enzyme de l'oxydation et que le N-éthylmaléimide (NEM) et l'ortho-phénanthroline sont des inhibiteurs puissants de l'enzyme.

La MGDG synthase d'épinard surexprimée présente les mêmes propriétés.

La *r*MGD A, purifiée par chromatographie d'hydroxyapatite est très active en présence de DTT. Si le DTT est éliminé par chromatographie sur une colonne Biogel P6-DG, la MGDG synthase perd 85 % de son activité, alors que l'addition de DTT maintient son activité.

Les fractions de *r*MGD A partiellement purifiée sont désalées par chromatographie sur une colonne (Pharmacia, colonne C10/40, 30 ml de gel) Biogel P6-DG (Bio-Rad), équilibrée sans DTT. Des aliquots (200 µl) des fractions sont incubés pendant 40 min à 25°C sous agitation douce en présence ou en l'absence de DTT. L'activité de galactosylation est alors mesurée comme précisé ci-dessus (voir exemple 3).

Les résultats obtenus sont résumés dans le Tableau I ci-après :

**Tableau I**

| Fraction purifiée sur hydroxyapatite | Activité (%) |
|---|---|
| Non désalée | 100 |
| Désalée | 15 |
| Désalée + DTT 1 mM | 75 |
| Désalée + DTT 10 mM | 65 |

L'activité est exprimée comme un pourcentage de l'activité contrôle (non désalée).

Le Tableau II montre que la rMGD A est très sensible au NEM et qu'une protection de l'activité est obtenue par préincubation en présence de DAG et/ou de PG.

**Tableau II**

| Préincubation | Incubation +/- NEM 150 µM | Incubation +/- DTT 10 mM | Réaction enzymatique | Activité (%) |
|---|---|---|---|---|
| (30 min) | (10 min) | (10 min) | | |
| DTT | - | - | +PG+DAG+UDP-gal | 100 |
| - | - | + | +PG+DAG+UDP-gal | 101 |
| - | - | - | +PG+DAG+UDP-gal | 37 |
| - | + | + | +PG+DAG+UDP-gal | 35 |
| DTT | + | - | +PG+DAG+UDP-gal | 108 |
| UDP-gal | + | + | +PG+DAG | 32 |
| PG | + | + | +DAG+UDP-gal | 56 |
| PG+DAG | + | + | +UDP-gal | 60 |

Pour obtenir les résultats résumés dans le Tableau II, la rMGD A est désalée par chromatographie sur une colonne (Pharmacia, colonne C10/40, 30 ml de gel) Biogel P6-DG (Bio-Rad), équilibrée sans DTT. Des aliquots (200 µl) des fractions sont incubés pendant 30 min à 25°C sous agitation douce, suivie d'une incubation de 10 min. en présence ou en l'absence de NEM 150 µM, puis d'une incubation de 10 min en présence ou en l'absence de DTT. L'activité de galactosylation est alors mesurée comme précisé ci-dessus (voir exemple 3).

L'activité est exprimée comme un pourcentage de l'activité contrôle , c'est-à-dire après incubation pendant 50 min. en présence de DTT 10 mM.

On observe également la *r*MGD surexprimée est inhibée par l'agent chélateur hydrophobe ortho-phénanthroline, comme le montre le Tableau III ci-après :

**Tableau III**

| Conditions | Autres additions | Activité (%) |
|---|---|---|
| Activité initiale (temps 0) | - | 100 |
| | | |
| Sans ortho-phénanthroline | - | 82 |
| | PG | 87 |
| | PG + DAG | 78 |
| | UDP-gal | 72 |
| | | |
| Avec ortho-phénanthroline | - | 43 |
| | PG | 27 |
| | PG + DAG | 92 |
| | UDP-gal | 17 |

L'inactivation de la rMGD A par l'ortho-phénanthroline est bloquée par le DAG, mais n'est pas affectée par l'UDP-gal.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Procédé de criblage et de sélection d'antiparasitaires et/ou d'herbicides, **caractérisé en ce qu'**il comprend :
- l'incubation d'une substance à tester avec une MGDG synthase ou avec une membrane plastidiale isolée de plante et
- la mesure de l'activité enzymatique de la MGDG synthase, après ladite incubation.

2. Procédé selon la revendication 1, **caractérisé en ce que** ladite MGDG synthase présente une activité spécifique initiale comprise entre 0,1 et 120 µmol de galactose incorporé/h/mg de protéine.

3. Procédé selon la revendication 1 ou la revendication 2, **caractérisé en ce que** l'incubation MGDG synthase/substance à tester est réalisée dans un milieu d'incubation contenant un tampon ajusté à un pH compris entre 6 et 9, en présence de détergents, d'un agent réducteur, de phosphatidylglycérol et d'un sel.

4. Procédé selon la revendication 3, **caractérisé en ce que** ledit milieu d'incubation contient de préférence 50 mM de MOPS-NaOH, pH 7,8, 4,5 mM de CHAPS, 1 mM de DTT, 1,3 mM de phosphatidylglycérol, 250 mM de KH₂PO₄/K₂HPO₄ et 250 mM de KCI.

5. Procédé selon l'une quelconque des revendications 1 à 4, **caractérisé en ce que** la MGDG synthase est d'origine végétale et est sélectionnée dans le groupe constitué par les MGDG synthase A et les MGDG synthase B purifiées ou recombinantes.

6. Procédé selon l'une quelconque des revendications 1 à 5, **caractérisé en ce que** ledit parasite apicomplexe est sélectionné dans le groupe constitué par *Plasmodium, Toxoplasma* et *Eimeria.*

## Claims

1. A method for screening and for selecting antiparasitic agents and/or herbicides, **characterized in that** it comprises:
- incubating a substance to be tested with an MGDG synthase or with a plastidial membrane isolated from a plant and
- measuring the enzymatic activity of the MGDG synthase, after said incubation.

2. The method as claimed in claim 1, **characterized in that** said MGDG synthase has an initial specific activity of between 0.1 and 120 µmol of galactose incorporated/h/mg of protein.

3. The method as claimed in claim 1 or claim 2, **characterized in that** the MGDG synthase/substance to be tested incubation is carried out in an incubation medium containing a buffer adjusted to a pH of between 6 and 9, in the presence of detergents, of a reducing agent, of phosphatidylglycerol and of a salt.

4. The method as claimed in claim 3, **characterized in that** incubation medium preferably contains 50 mM of MOPS-NaOH, pH 7.8, 4.5 mM of CHAPS, 1 mM of DTT, 1.3 mM of phosphatidylglycerol, 250 mM of KH₂PO₄/K₂HPO₄ and, 250 mM of KCI.

5. The method according to any one of claims 1 to 4, **characterized in that** the MGDG synthase is of plant origin and is selected from the group consisting of the purified or recombinant MGDG synthases A and MGDG synthases B.

6. The method as claimed in any one of claims 1 to 5, **characterized in that** said apicomplex parasite is selected from the group consisting of *Plasmodium, Toxoplasma* and *Eimeria.*

## Patentansprüche

1. Verfahren zum Screening und zur Selektion von Antiparasitika und/oder Herbiziden, **dadurch gekennzeichnet, dass** es umfasst:
Inkubation einer zu testenden Substanz mit einer MGDG-Synthase oder mit einer isolierten Pflanzenplastidenmembran und
Messen der Enzymaktivität der MGDG-Synthase nach der Inkubation.

2. Verfahren nach Anspruch 1, **dadurch gekennzeichnet, dass** die MGDG-Synthase eine spezifische Anfangsaktivität zwischen 0,1 und 120 µmol eingebaute Galaktose/h/mg Protein hat.

3. Verfahren nach Anspruch 1 oder Anspruch 2, **dadurch gekennzeichnet, dass** die Inkubation von MGDG-Synthase/zu testender Substanz in einem Inkubationsmedium, das einen auf einen pH zwischen 6 und 9 eingestellten Puffer enthält, in Gegenwart von Detergenzien, einem Reduktionsmittel, Phosphatidylglycerin und einem Salz erfolgt.

4. Verfahren nach Anspruch 3, **dadurch gekennzeichnet, dass** das Inkubationsmedium vorzugsweise 50 mM MOPS-NaOH, pH 7,8, 4,5 mM CHAPS, 1 mM DTT, 1,3 mM Phosphatidylglycerin, 250 mM KH₂PO₄/K₂HPO₄ und 250 mM KCI enthält.

5. Verfahren nach einem der Ansprüche 1 bis 4, **dadurch gekennzeichnet, dass** die MGDG-Synthase pflanzlichen Ursprungs ist und ausgewählt ist aus der Gruppe bestehend aus gereinigter oder rekombinanter MGDG-Synthase A und MGDG-Synthase B.

6. Verfahren nach einem der Ansprüche 1 bis 5, **dadurch gekennzeichnet, dass** der Apikalkomplexparasit ausgewählt ist aus der Gruppe bestehend aus *Plasmodium, Toxoplasma* und *Eimeria.*


### LISTAGE DE SEQUENCE

<110> COMMISSARIAT A L'ENERGIE ATOMIQUE
   MARECHAL Eric
   BLOCK Maryse
   JOYARD Jacques
   DOUCE Roland
<120> PROCEDE DE CRIBLAGE ET DE SELECTION D'ANTIPARASITAIRES APICOMPLEXES ET/OU D'HERBICIDES ET SES APPLICATIONS.
<130> BLOcp263/54P
<140>
   <141>
<160> 7
<170> Patent In Vers. 2.0
<210> 1
   <211> 23
   <212> ADN
   <213> Spinacia oleracea
<400> 1
   ctcatttgaa gggcagtagc acc 23
<210> 2
   <211> 27
   <212> ADN
   <213> Spinacia oleracea
<400> 2
   cacacaatat ttccaatgta tacccac 27
<210> 3
   <211> 25
   <212> ADN
   <213> Spinacia oleracea
<400> 3
   gattatcatt tcccctcgcc ctgcc 25
<210> 4
   <211> 26
   <212> ADN
   <213> Spinacia oleracea
<400> 4
   ggagcatatg ggggtgagtg ataatg 26
<210> 5
   <211> 28
   <212> ADN
   <213> Spinacia oleracea
<400> 5
   gttctggatc ctcaagcagc acaagagt 28
<210> 6
   <211> 28
   <212> ADN
   <213> Spinacia oleracea
<400> 6
   cttcacatat gcttaattcc ggggagag 28
<210> 7
   <211> 28
   <212> ADN
   <213> Spinacia oleracea
<400> 7
   gttctggatc ctcaagcagc accgagta 28